# EUROPEAN PATENT SPECIFICATION

(11) **EP 2 548 900 B1**
(45) Date of publication and mention of the grant of the patent: **02.08.2017**
(21) Application number: 11755738.9
(22) Date of filing: 18.03.2011
(51) Int. Cl.: C08F 26/06, A61K 47/32

(54) **CATIONIC NANOGELS FOR BIOTECHNOLOGICAL USES**
KATIONISCHE NANOGELE FÜR BIOTECHNOLOGISCHE ANWENDUNGEN
NANOGELS CATIONIQUES POUR APPLICATIONS BIOTECHNOLOGIQUES

(30) Priority: 18.03.2010 ES 201030399
(43) Date of publication of application: 23.01.2013
(73) Proprietor: Universidad del Pais Vasco, 48940 Leioa Vizcaya (ES); Eindhoven University Of Technology, 5600 MB Eindhoven (NL); Universidad De Granada, 180071 Armilla (Granada) (ES)
(72) Inventor: FORCADA GARCÍA, Jacqueline, E-48940 Leioa - Vizcaya (ES); IMAZ MAKAZAGA, Ainara, E-48940 Leioa - Vizcaya (ES); SAN MARTÍN SÁEZ, Andoni, E-48940 Leioa - Vizcaya (ES); RAMOS JULIÁN, José, E-18071 Armilla - Granada (ES); VAN HERK, Alex, M., NL-5600 MB Eindhoven (NL); HEUTS, Johannes A., P., NL-5600 MB Eindhoven (NL)
(74) Representative: ABG Patentes, S.L.
(86) International application number: PCT/ES2011/070188
(87) International publication number: WO 2011/113989

(56) References cited:
- WO-A2-2008/155282
- ES-A1- 2 319 042
- BOYKO V ET AL: "Thermo-sensitive poly(N-vinylcaprolactam-co-acetoacetoxyeth yl methacrylate) microgels: 1-synthesis and characterization", POLYMER, ELSEVIER SCIENCE PUBLISHERS B.V, GB, vol. 44, no. 26, 1 December 2003 (2003-12-01), pages 7821-7827, XP027140896, ISSN: 0032-3861 [retrieved on 2003-11-19]
- PICH, ANDRIJ ET AL.: 'Biocompatible hybrid nanogels' SMALL vol. 4, no. 12, 2008, pages 2171 - 2175, XP055124708

## Description

### Field of the Invention

The present invention relates to thermo-sensitive, biocompatible cationic nanogels as well as to a method for their production and to pharmaceutical compositions comprising said nanogels. Among other applications, the nanogels of the invention can be used as vehicles for carrying and releasing biologically active agents.

### Background of the Invention

Over the last few decades, aqueous polymer dispersions prepared by means of polymerization processes in a dispersed medium leading to the production of polymer particles with sizes comprised within the colloidal range have gained an increasing interest from both the academic and industrial points of view (Forcada J. and Hidalgo, R., Curr. Org. Chem. 2005, 9, 1067-1084). These nanoparticles are used in a large variety of applications ranging from adhesives, coatings based on water, paper, textile, additives, flocculants, etc. They are also suitable as fine polymer material or polymer material with high added value for medical diagnostic tests, antibody purification, drug release systems and as calibration material.

Nanogels are one particular type of these colloidal systems. Nanogels are cross-linked colloidal particles which may swell by absorbing large amounts of solvent, but they do not dissolve due to the structure of the physically or chemically cross-linked polymer network forming them.

Nanogels exhibit a behavior ranging from that of a polymer solution (swollen state) to that of a "hard" particle (collapsed state). They can respond to physical stimuli (temperature, ionic strength, magnetic fields, electric fields, etc.), chemical stimuli (pH, ions and specific molecules, etc.) and biochemical stimuli (enzyme substrates, affinity ligands, etc.). Among the foregoing, temperature is the most widely studied since it is a very effective stimulus in many applications. The nanogels capable of undergoing a volumetric phase change upon changing the temperature of the medium in which they are dispersed, or temperature sensitive nanogels, are very interesting in those biotechnological applications in which an active ingredient, a molecule or a material is to be dosed in media in which the main variable is temperature. Furthermore, it has been found that nanogel particles are capable of responding to stimuli faster than their macroscopic gel counterparts, i.e., hydrogels, due to their small size [Couvreur P. et al., Polymeric Nanoparticles and Microspheres, Guiot, P.; Couvreur, P., ed., CRC Press, Boca Raton, Fla., 27-93, 1986]. Therefore, the size of the particles forming the nanogel is a very important parameter since it controls the release system efficacy.

Nanogels used as vehicles for delivering and releasing molecules or biologically active material must remain chemically unaltered and be pharmacologically stable during their transit from the administration site to the target where they are going to exert their effect. Likewise, the characteristics of the vehicle must be such that it has to be not only compatible with the path that it has to go through, but also with the dosing site, i.e., it has to be "intelligent" and must release the active ingredient at the precise moment when it has reached the target and allowing the bioavailability thereof. On the other hand, the carrier or vehicle must not only maintain its characteristics and properties in an aqueous medium but must also be capable of showing its properties again if it needs to be stored in a dry (lyophilized) state for the application and/or transport. In other words, once redispersed, it must again show its properties and behavior with respect to temperature changes.

The most commonly used family of polymers in the synthesis of microgels and nanogels sensitive to the conditions of the medium is temperature sensitive poly(alkylacrylamides), more specifically poly(N-isopropylacrylamide) (PNIPAM). However, the toxicity of poly(alkylacrylamides) prevents their use in biomedical applications. Despite this, there are many articles and patent documents published throughout recent years regarding such systems. Cationic nanogels have particularly been described. In this sense, Moselhy, J. et al. (Int. J. Nanomedicine 2009, 4, 153-164) describe thermo-sensitive cationic nanogels based on a polymer network of N-isopropylacrylamide (NIPAM) or on a copolymer thereof with 2-(dimethylamino) ethyl methacrylate (DMAEMA) and their potential use as DNA release vehicles; Eke, I. et al. (Colloids and Surfaces A: Physicochem. Eng. Aspects 2006, 279, 247-253) describe both pH and temperature sensitive cationic microgels synthesized from N-isopropylacrylamide and N-3-dimethyl-aminopropylmethacrylamide (DMAPM) and their use in the development of new surfaces related to biotechnological applications; WO 2006/102762 proposes a technique for grafting boronic acid groups into PNIPAM microgels with carboxyl groups, which are used for the controlled release of insulin; Nolan, C.M. et al. (Biomacromolecules 2006, 7(11), 2918-2922) also investigate the release of insulin from PNIPAM microgels using variable-temperature ¹H NMR; Sahiner, N. et al. (Colloid Polym. Sci. 2006, 284, 1121-1129) refer to obtaining pH sensitive cationic micro- and nanogels by means of polymerizing (3-acrylamidopropyl)-trimethylammonium chloride (APTMAC) and their numerous applications within the field of biomedicine and biotechnology, particularly their use as vehicles for the controlled release of active molecules.

Temperature sensitive biocompatible monomers include *N-*vinylcaprolactam (VCL) [Vihola, H. et al. Biomaterials 2005, 26, 3055-3064], which is a water soluble monomer. Its corresponding polymer, poly N-vinylcaprolactam (PVCL), combines very useful and important properties because, in addition to being biocompatible, it has a phase transition in the physiological temperature (32-38°C) region. This combination of properties allows considering it as a material suitable for designing biomedical devices and for use in drug release systems. Therefore, document WO 2008/145612, for example, describes obtaining anionic *N*-vinylcaprolactam microgels suitable as an active ingredient release system undergoing a progressive volumetric phase change from the swollen state to the collapsed state as the temperature of the medium in which they are dispersed increases.

Nevertheless, none of the cationic or anionic nanogels described in the state of the art shows an immediate response to a temperature change in the phase transition region, let alone said response involving a volumetric change such that the nanogel changes from the collapsed state to the swollen state as the temperature increases.

### Brief Description of the Invention

The authors of the present invention have observed that a cationic nanogel made up of a cross-linked polymer network of N-vinylcaprolactam monomer units, obtained by polymerization in a dispersed medium in the presence of a cationic or non-ionic emulsifier and a cationic initiator, undergoes an instantaneous volumetric change at the phase transition temperature of the polymer (32-38°C), changing from the collapsed state to the swollen state as the temperature increases, which allows it to immediately release an active ingredient within the physiological temperature region.

Therefore, in a first aspect, the invention relates to a biocompatible cationic nanogel comprising a polymer network, said polymer network comprising polymer units interconnected with one another through a cross-linking agent, wherein said polymer network can be obtained by polymerizing N-vinylcaprolactam and a cross-linking agent in a dispersed medium, in the presence of a cationic initiator and a cationic or non-ionic emulsifier.

An additional aspect of the present invention comprises a pharmaceutical composition comprising a biocompatible cationic nanogel as defined above, and a pharmaceutically acceptable excipient, vehicle or adjuvant.

The last aspect of the present invention relates to a method for obtaining a biocompatible cationic nanogel as defined above, which comprises polymerizing a composition comprising N-vinylcaprolactam and a cross-linking agent in a dispersed medium, in the presence of a cationic initiator and a cationic or non-ionic emulsifier.

### Brief Description of the Drawings

Figure 1 shows the transmission electron microscopy images taken at approximately 22°C and 60°C clearly showing that the nanogels of the invention are collapsed at temperatures below the phase transition temperature of the polymer and swollen at temperatures above said temperature.
Figure 2 is a graph showing the variation of the average hydrodynamic diameter with respect to the temperature experienced by the nanogels made up of an N-vinylcaprolactam (VCL) and styrene (St) copolymer [a) 90% VCL/10% St; b) 70% VCL/30% St; c) 50% VCL/50% St]
Figure 3 is a graph showing the variation of the average hydrodynamic diameter with respect to the temperature experienced by nanogels made up of an N-vinylcaprolactam (VCL) and methyl methacrylate (MMA) copolymer [a) 90% VCL/10% MMA; b) 80% VCL/20% MMA; c) 70% VCL/30% MMA; d) 50% VCL/50% MMA].
Figure 4 is a graph showing the variation of the average hydrodynamic diameter with respect to the temperature experienced by nanogels of the invention for different concentrations of emulsifier S7A13.
Figure 5 is a graph showing the variation of the average hydrodynamic diameter with respect to the temperature experienced by nanogels of the invention for different concentrations of initiator: a) 1% by weight of initiator ADIBA (E20I1); b) 0.5% by weight of initiator ADIBA (E20I0.5); c) 0.3% by weight of initiator ADIBA (E20I0.3).
Figure 6 is a graph showing the variation of the average hydrodynamic diameter with respect to the temperature experienced by nanogels of the invention with different emulsifiers: a) S7A13 (E20); b) HDTAB (H20); c) DTAB (D20); d) Tween 20 (NI20).
Figure 7 is a graph showing the variation of the average hydrodynamic diameter with respect to the temperature experienced by the cationic nanogel E20 after its lyophilization and resuspension.

### Detailed Description of the Invention

In one aspect, the invention relates to a biocompatible cationic nanogel, hereinafter nanogel of the invention, comprising a polymer network, said polymer network comprising polymer units interconnected with one another through a cross-linking agent, wherein said polymer network can be obtained by polymerizing *N*-vinylcaprolactam and a cross-linking agent in a dispersed medium, in the presence of a cationic initiator and a cationic or non-ionic emulsifier.

As used herein, the term "nanogel" refers to a three-dimensional hydrogel particle the mean diameter of which is less than 1 µm, i.e., a diameter comprised between 1 and 999 nm. Likewise, a "hydrogel" is a three-dimensional macromolecular network which swells in water and is formed from a cross-linked polymer (e.g., polymer units interconnected with one another by means of a cross-linking agent).

The cationic nature of the nanogel of the invention results from using a cationic initiator in the polymerization process.

If desired, said nanogel of the invention can further contain at least one biologically active agent, giving rise to a biocompatible nanogel loaded with a biologically active agent which can be used for carrying, delivering and/or dosing biologically active agents to the site of interest. The biocompatible nanogels loaded or not loaded with biologically active agents provided by this invention are sensitive to the temperature changes of the medium in which they are dispersed, and they can swell and shrink (i.e., change size), thus responding to temperature stimuli.

In particular, the main advantage of the thermo-sensitive, biocompatible cationic nanogels of the present invention with respect to other nanogels of the state of the art is that in addition to being biocompatible, they undergo an instantaneous volumetric change at the phase transition temperature of the polymer, changing from the collapsed state to the swollen state as the temperature increases, thus allowing the immediate release of the active ingredient within the physiological temperature region.

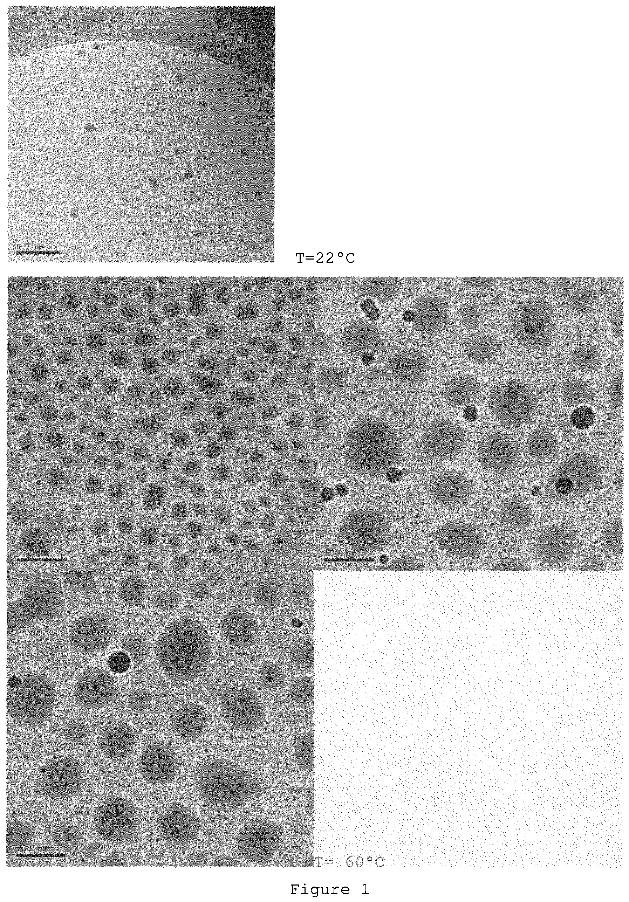

Figure 1 shows the transmission electron microscopy images for a cationic nanogel according to the present invention taken at approximately 22°C and 60°C, in which it can be observed how the nanogel particles are swollen at temperatures higher than the phase transition temperature of the main polymer making up the polymer network and collapsed below said temperature. It furthermore shows that the nanogel does not increase in size as a result of the agglomeration or aggregation of several particles but rather as a result of the swelling of the individual nanogel particles.

The biocompatible nanogel nanoparticles loaded or not loaded with a biologically active agent provided by this invention can be lyophilized and then resuspended in an aqueous medium, preserving their properties and thermo-sensitivity. Therefore, the nanogels provided by this invention are biocompatible materials, useful as carriers for biologically active agents, capable of absorbing (encapsulating/uptake) both biologically active hydrophobic and hydrophilic agents and suitably dosing them since they respond to temperature stimuli of the medium in which they are dispersed.

### N-vinylcaprolactam (VCL)

N-vinylcaprolactam (VCL) is a compound with the following chemical formula

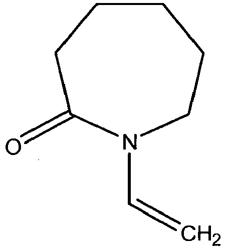

VCL is an amphiphilic monomer since it contains hydrophilic groups (amide) and hydrophobic groups (vinyl group and alkyl groups of the lactam ring). VCL is partially water soluble, its solubility being 8.5% by weight. The water solubility of the corresponding homopolymer, poly(*N*-vinylcaprolactam) (PVCL) varies with temperature. The presence of the hydrophobic and hydrophilic groups means that repulsive and attractive forces co-exist. The balance of these forces determines the polymer solubility.

Some polymers are water soluble at low temperature, being separated from the solution and forming a separate phase as the temperature increases above a value known as "lower critical solution temperature" (LCST), also called "phase change critical temperature". In the case of PVCL, this phase change critical temperature is in the range comprised between 32 and 38°C, i.e., close to the physiological temperature.

PVCL further has an additional characteristic, its biocompatibility, making it susceptible to being used in biomedical applications. During the development of this invention, it has been observed that the synthesis of said polymer must not be carried out at acid pH for the purpose of preventing VCL hydrolysis.

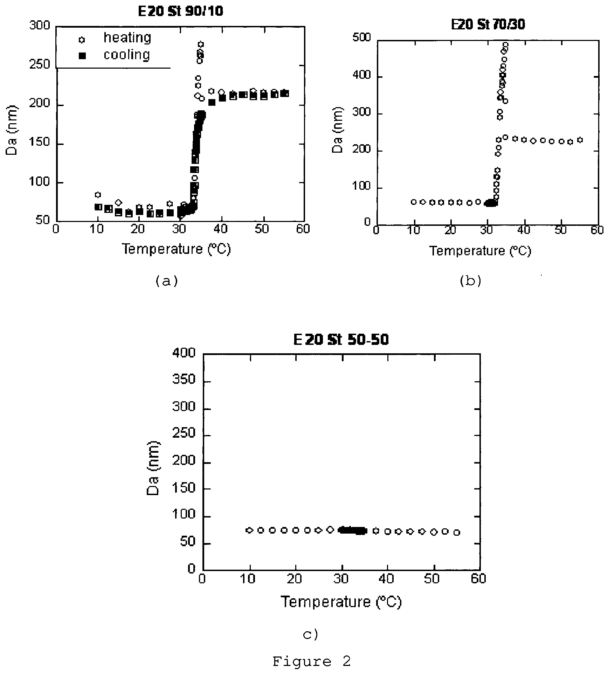

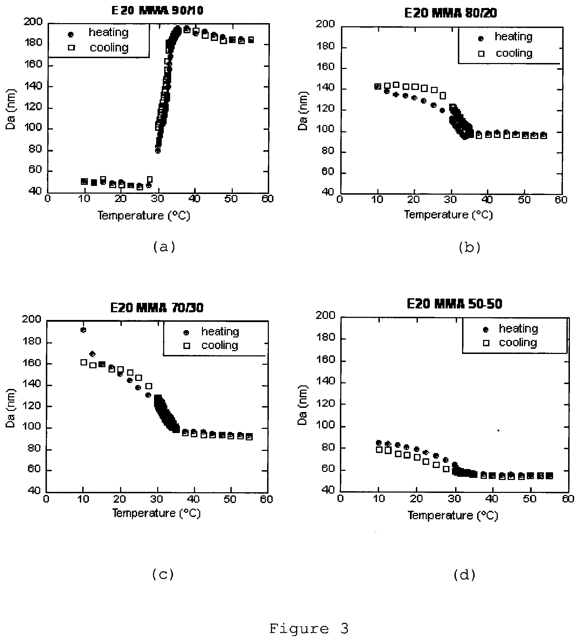

The authors of the present invention have observed that the copolymerization of VCL with other monomers causes a significant change in the behavior of the polymer with respect to temperature. Therefore, when VCL is copolymerized with styrene in a ratio of 50:50, the nanoparticles are not temperature sensitive and behave like conventional latex (see Figure 2). Copolymerization with methyl methacrylate (MMA) in turn provides particles which behave like anionic nanogels, i.e., they undergo the volumetric change expected for PVCL, changing from the swollen state to the collapsed state as the temperature increases (see Figure 3). This is because methyl methacrylate is pH sensitive and when the pH of the medium exceeds the pKa of the carboxyl group from MMA hydrolysis, said group ionizes providing negative charges to the nanogel of the invention.

The concentration of VCL with respect to the total concentration of the formulation (recipe) comprising the components necessary for obtaining the polymer units interconnected with one another through a cross-linking agent making up the polymer network of the biocompatible nanogel of the invention can vary within a wide range; specifically, between the concentration of VCL which does not form a macroscopic gel when polymerized (maximum concentration) and the concentration of VCL which allows obtaining the final desired diameter of the biocompatible nanogel of the invention when polymerized (minimum concentration). In a particular embodiment, the concentration of VCL is comprised between 0.5-3% by weight with respect to the total weight of the recipe. In an even more particular embodiment, the concentration of VCL is approximately 1% by weight with respect to the total weight of the recipe.

### Cross-linking agent

According to the present invention, the component "cross-linking agent" is a difunctional monomer comprising at least two vinyl groups.

In a specific embodiment, said cross-linking agent is a dextran with two or more vinyl groups.

In another specific embodiment, said cross-linking agent is *N,N'*-methylenebisacrylamide (BA).

In another particular embodiment, said cross-linking agent is a difunctional monomer which, in addition to two vinyl groups, comprises at least one ethylene glycol unit. Non-limiting illustrative examples of said cross-linking agent include a difunctional monomer of general formula 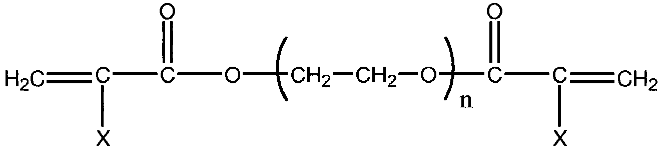 wherein
X is hydrogen or methyl, and
n is an integer comprised between 1 and 90.

In a specific embodiment, when "n" is 1 and "X" is hydrogen, the cross-linking agent is ethylene glycol diacrylate (EGDA), and when "n" is greater than 1 and "X" is hydrogen, the cross-linking agent is poly(ethylene glycol diacrylate) (PEGDA).

In another specific embodiment, when "n" is 1 and "X" is methyl, the cross-linking agent is ethylene glycol dimethacrylate (EGDMA), and when "n" is greater than 1 and "X" is methyl, the cross-linking agent is poly(ethylene glycol dimethacrylate) (PEGDMA).

In a particular embodiment, the cross-linking agent is a difunctional monomer selected from the group consisting of a dextran with two or more vinyl groups, *N,N'-*methylenebisacrylamide (BA), EGDA, PEGDA, EGDMA, PEGDMA and mixtures thereof.

Some of said cross-linking agents have the characteristic where, in addition to the oxygen atoms of the carbonyl groups, the oxygen atoms present in the ethylene glycol units can form hydrogen bonds with carboxyl groups.

The concentration of cross-linking agent with respect to the VCL present in the formulation (recipe) comprising the components necessary for obtaining the polymer units interconnected with one another through said cross-linking agent making up the polymer network of the biocompatible nanogel of the invention can vary within a wide range, depending on the desired diameter of the biocompatible nanogel of the invention (at temperatures higher or lower than the LCST); nevertheless, in a particular embodiment, the concentration of cross-linking agent present in said recipe is comprised between 2% and 10% by weight with respect to VCL; in an even more particular embodiment, the concentration of cross-linking agent present in said recipe is approximately 8% by weight with respect to VCL.

### Cationic initiator

According to the present invention, the component "cationic initiator" refers to a compound which decomposes by means of thermal activation generating positively charged reactive species which start the radical polymerization process. As a result, the initiator used in the invention is a cationic thermal initiator.

The cationic nature of the initiator is of vital importance in nanogel preparation given that it is the compound which will confer the positive charge to the nanogel, the charge being what allows the nanogel nanoparticles to undergo an instantaneous volumetric change in the phase transition temperature and allows said change to involve the transition from the collapsed state to the swollen state as the temperature increases.

Although virtually any suitable initiator capable of decomposing and generating positive species could be used, in a particular embodiment, said initiator is selected from the group consisting of 2,2- azobis(N, N'-dimethylene isobutyramidine) dihydrochloride (ADIBA), 2,2'-azobis[2-(2-imidazolin-2-yl)propane] disulfate dihydrate, 2,2'-azobisisobutyramidine dihydrochloride (AIBA), 2,2'-azobis{2-[1-(2-hydroxyethyl)-2-imidazolin-2-yl]propane} dihydrochloride and 2,2'-azobis(1-imino-1-pyrrolidino-2-ethylpropane) dihydrochloride.

The respective formulas of these initiators are shown below:

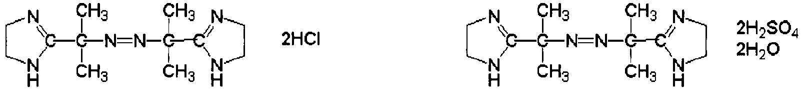

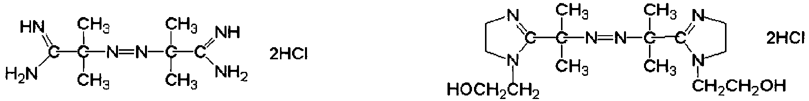

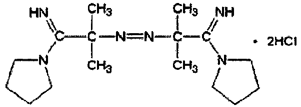

According to a preferred embodiment, the initiator used is 2,2-azobis(N,N'-dimethylene isobutyramidine) dihydrochloride (ADIBA), which decomposes thermally providing reactive cationic species which allow starting the polymerization and cross-linking of VCL via radical.

The concentration of initiator which can be used for putting said method into practice can vary within a wide range; nevertheless, in a particular embodiment, the concentration of initiator is comprised between 0.3% and 1.5% by weight with respect to VCL; in even more particular embodiments, the concentration of initiator present in said recipe ranges between 0.3 and 1.2% by weight with respect to VCL.

### Emulsifier

Emulsifier confers colloidal stability to the cationic nanogels of the invention. It covers the polymer network making up the nanogel nanoparticles. According to the present invention, the emulsifier can be both cationic and non-ionic emulsifier.

Although virtually any suitable cationic or non-ionic emulsifier could be used, in a particular embodiment, the emulsifier is a non-ionic emulsifier, preferably Tween 20. The formula corresponding to this compound is shown below:

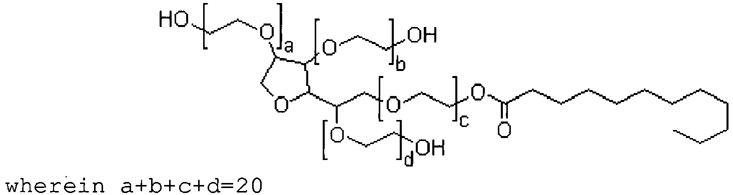

In another particular embodiment, said emulsifier is a cationic emulsifier. More particularly, said cationic emulsifier is an alkyltrimethylammonium halide. The alkyltrimethylammonium halide is preferably selected from hexadecyltrimethylammonium bromide (HDTAB) and dodecyltrimethylammonium bromide (DTAB), respectively having the following formulas:

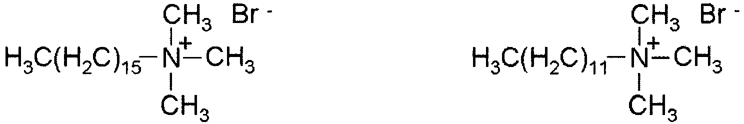

In a still more preferred embodiment, the cationic emulsifier is a quaternary ammonium block copolymer, this being understood as a block copolymer having a quaternary ammonium terminal group, such as for example, a styrene and 2-(dimethylamino) ethyl methacrylate block copolymer of formula: 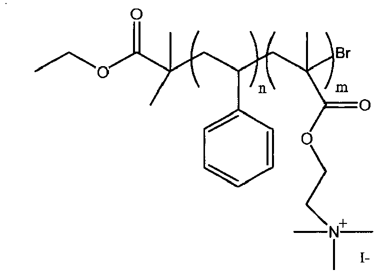 wherein n and m are integers comprised between 5 and 50.

Among the compounds corresponding to this formula, the use of the styrene and 2-(dimethylamino) ethyl methacrylate block copolymer defined in the preceding formula in which n=7 and m=13 is preferred. Throughout this specification said compound is called S7A13.

These block copolymers can be synthesized by any method known by a person skilled in the art. Nevertheless, in a particular embodiment, the synthesis of said copolymer is carried out in three steps. A first step of polymerizing the polystyrene chain which is carried out in the presence of an initiator and is catalyzed by a copper complex, such as for example CuBr₂/tris[2-(dimethylamino)ethyl]amine; a second step of elongating the polystyrene chain with 2-(dimethylamino) ethyl methacrylate (also called DMAEMA) which is performed by reacting the polystyrene with DMAEMA in the presence of the mentioned copper complex; and a third step of quaternizing the amino group of methacrylate by reacting with an alkyl halide, such as methyl iodide for example. A detailed example of the synthesis of this block copolymer is detailed in Example 1, wherein n=7 and m=13.

The concentration of emulsifier which can be used for putting the method of the invention into practice can vary within a wide range; nevertheless, in a particular embodiment, the concentration of emulsifier is comprised between 0.2% and 25% by weight with respect to VCL; in an even more particular embodiment, the concentration of emulsifier present in said recipe can range between 0.3 and 20% by weight with respect to VCL.

### Obtaining the cationic nanogel object of the invention

The biocompatible cationic nanogel of the invention is obtained as a result of cross-linking PVCL polymer units interconnected with one another through the cross-linking agent, generating a polymer network. Said polymer network can be obtained by means of a method of polymerization of the main monomer (VCL) in the presence of said cross-linking agent in a dispersed medium, such as emulsion polymerization. In the context of the invention, the emulsion polymerization of said monomers also comprises the use of a cationic initiator and of a cationic or non-ionic emulsifier. The polymerization of a monomer, as well as the copolymerization of two or more different monomers in a dispersed (heterogeneous) medium is known by the persons skilled in the art. In general, in such method of polymerization, water (e.g., distilled water) is used as the continuous reaction medium.

The polymerization reaction can be carried out at a temperature comprised within a wide range; nevertheless, it is very important to take into account the phase change critical temperature (LCST) of the polymer units making up the polymer network of the biocompatible nanogel of the invention when choosing the reaction temperature. In a particular embodiment, the polymerization reaction is carried out at a temperature greater than the LCST of the different polymer units forming part of the biocompatible nanogels of the invention. In a specific embodiment, the polymerization reaction is carried out at a temperature equal to or higher than 60°C.

Likewise, the stirring speed of the formulation (recipe) containing the components necessary for generating the biocompatible nanogel of the invention by means of polymerization in a dispersed medium as mentioned above can vary within a wide range; nevertheless, the stirring speed must be high enough in order to achieve a homogenous mixture of the dispersion (reaction mixture) when synthesizing the biocompatible nanogels of the invention. In a particular embodiment, the stirring speed is equal to or greater than 200 rpm; in another particular embodiment, the stirring speed is comprised between 200 and 400 rpm, for example approximately 300 rpm.

The polymerization reaction can be carried out in a reactor discontinuously (or in batches) or semi-continuously.

The technical features of the biocompatible nanogels of the invention will depend, among other factors, on the nature and concentration of VCL used, cross-linking agent, emulsifier, initiator, and reaction temperature. However, by way of non-limiting illustration, the values of some technical features of said biocompatible nanogels of the invention can be indicated in ranges:
- Diameter of collapsed particle (15°C): 30-60 nm
- Diameter of swollen particle (55°C): 150-800 nm
- Phase transition temperature: 32.5°C - 38°C

The cationic nanogels of the invention may both be dispersed in water and lyophilized. Once lyophilized, if they are resuspended in an aqueous medium, they maintain their properties and capacity to swell/collapse with respect to temperature changes of the dispersion medium.

### Biocompatible nanogels loaded with a biologically active agent

As mentioned above, the cationic biocompatible nanogels of the invention are capable of uptaking or encapsulating biologically active agents. Therefore, in a particular embodiment, the biocompatible nanogel of the invention further comprises a biologically active agent. In general, said biologically active agent can be inside the biocompatible nanogel of the invention; nevertheless, sometimes the biologically active agent can also be bound to or adsorbed on the outer surface of said nanogel.

The cationic biocompatible nanogels loaded with biologically active agents provided by this invention are in the form of nanoparticles which are sensitive to the temperature changes of the medium in which they are dispersed and can swell and collapse (i.e., change in size), thus responding to temperature stimuli. They particularly undergo an instantaneous volumetric change in the phase temperature of the main polymer making up the polymer network, changing from a collapsed state to a swollen state as the temperature increases.

The biocompatible nanogel nanoparticles loaded or not loaded with a biologically active agent provided by this invention can be lyophilized and then resuspended in an aqueous medium, preserving their properties and sensitivities.

The temperature sensitive biocompatible nanogels of the present invention have a number of advantages with respect to other "intelligent" materials which cannot be used in drug dosing because they do not meet the biocompatibility condition. In these novel nanogels, the driving force controlling the absorption and subsequent dosing of the biologically active agent has a certain covalent character, which entails an additional advantage both in carrying the biologically active agent and in dosing it at the chosen site.

On the other hand, unlike the anionic nanogels of PVCL, the cationic nanogels of the invention allow immediately releasing the active ingredient due to the instantaneous volumetric change they undergo.

As used herein, the term "biologically active agent" refers to any substance which is administered to a subject, preferably a human being, for prophylactic or therapeutic purposes; i.e., any substance which can be used in treating, healing, preventing or diagnosing a disease or for improving the physical and mental well-being of humans and animals, for example, drugs, antigens, allergens, etc.

The biocompatible nanogel of the invention or the biocompatible nanogel nanoparticles of the invention can incorporate one or more biologically active agents regardless of the solubility characteristics thereof.

The chemical nature of the biologically active agent can vary within a wide range from small molecules to macromolecular compounds (peptides, polynucleotides, etc.). In a particular embodiment, said biologically active agent is a peptide or a protein. In another particular embodiment, said biologically active agent is a nucleoside, a nucleotide, an oligonucleotide, a polynucleotide or a nucleic acid (e.g., RNA or DNA). In another particular embodiment, said biologically active agent is a small (organic or inorganic) molecule; these molecules are generally obtained by semi-synthetic or chemical synthesis methods, or alternatively they are isolated from their sources. In a specific embodiment, said small (organic or inorganic) molecule has a relatively low molecular weight, generally equal to or less than 5,000, typically equal to or less 2,500, advantageously equal to or less 1,500. Many therapeutic active ingredients (drugs) have these characteristics and can therefore be used for putting the present invention into practice.

The biocompatible nanogels of the invention (and, consequently, the biocompatible nanogel nanoparticles of the invention) can be used as vehicles for the administration of both hydrophilic and hydrophobic substances. Non-limiting illustrative examples of biologically active agents (defined by therapeutic groups) encapsulated inside said nanogels and nanoparticles which can be used include: anti-carcinogenic substances, antibiotics, immunosuppressants, anti-viral substances, enzyme inhibitors, neurotoxins, opioids, hypnotics, antihistamines, tranquilizers, antiepileptic agents, muscle relaxants and anti-Parkinson substances, antispasmodic and muscle contraction agents, miotics and anticholinergics, antiglaucoma compounds, antiparasitic and/or antiprotozoal compounds, antihypertensives, analgesics, antipyretics and antiinflammatories, local anesthetics, prostaglandins, antidepressants, antipsychotic substances, antiemetics, neurotransmitters, proteins, cell response modifiers and vaccines.

Therefore, the nanogels provided by this invention are biocompatible materials useful as carriers of biologically active agents, capable of absorbing (encapsulating/uptake) both hydrophobic and hydrophilic biologically active agents, and suitably dosing them since they respond to the temperature stimuli of the medium in which they are dispersed.

The biocompatible nanogels of the invention can uptake or encapsulate the biologically active agent by conventional methods. In a particular embodiment, the polymerization reaction in a dispersed medium for producing the biocompatible nanogel of the invention can be carried out in the presence of the biologically active agent for the purpose of encapsulating said agent therein. Alternatively, the method for obtaining the biocompatible nanogel loaded with a biologically active agent provided by this invention comprises contacting a dispersion comprising a biocompatible nanogel of the invention with a solution comprising said biologically active agent (or agents) to be encapsulated.

More specifically, in a particular embodiment, a dispersion comprising the biocompatible nanogels of the invention (or nanoparticles based on said nanogels) is mixed with a solution of the biologically active agent to be uptaken, at a specific pH; the resulting solution is stirred for a suitable time period and the nanogels of the invention loaded with the biologically active agent are separated by conventional methods, such as centrifugation (e.g., at 10,000-15,000 rpm and 20°C). After separating the loaded nanogels the supernatant is analyzed by conventional methods, e.g., by means of spectrophotometry. The amount of biologically active agent uptaken or absorbed can be calculated by knowing the concentration of biologically active agent which has been used and the concentration of biologically active agent which remained in the supernatant.

### Pharmaceutical compositions

In another aspect, the invention relates to a pharmaceutical composition comprising a biocompatible nanogel loaded with a biologically active agent provided by this invention and a pharmaceutically acceptable excipient, vehicle or adjuvant.

Non-limiting illustrative examples of pharmaceutical compositions include any composition (solid or semi-solid) intended for oral, buccal, sublingual, topical, ocular, intranasal, pulmonary, rectal, vaginal, parenteral, topical administration, etc. In a particular embodiment, the pharmaceutical composition is administered orally due to its biocompatibility. The biocompatible nanogels of the invention are "intelligent" materials which provide a more controlled release of the biologically active agent and protect said biologically active agents during release, their bioavailability thus being able to be controlled in a uniform and constant manner. The reversibility of the swelling properties of these nanogels makes these materials the excellent vehicles for carrying both small biologically active agents and new macromolecular drugs (for example, peptides) and other therapeutic products.

The pharmaceutical compositions described will comprise the excipients suitable for each formulation. In the case of oral formulations in the form of tablets or capsules, for example, binding agents, disintegrants, lubricants, loading agents, enteric coating, etc., will be included if necessary. Solid oral formulations are prepared in a conventional manner by mixing, dry or wet granulation and by incorporating the biocompatible nanogel of the invention loaded with the biologically active agent. The pharmaceutical compositions can also be adapted for parenteral administration, for example in the form of sterile solutions, suspensions or lyophilized products, in the suitable dosage form; in this case, said pharmaceutical compositions will include the suitable excipients, such as buffers, surfactants, etc. In any case, the excipients will be chosen depending on the pharmaceutical dosage form selected. A review of the different pharmaceutical dosage forms of drugs and of their preparation can be found in the book "Tratado de Farmacia Galénica", by C. Faulí i Trillo, 10 Edition, 1993, Luzán 5, S.A. de Ediciones.

The proportion of the biologically active agent incorporated in the biocompatible nanogels of the invention can vary within a wide range, for example it can be up to 50% by weight with respect to the total weight of the nanoparticles. Nevertheless, in each case the suitable proportion will depend on the biologically active molecules incorporated.

The invention is described below by means of several illustrative non-limiting examples of the invention.

### Examples

### Materials

The main monomer: *N*-vinylcaprolactam (VCL, Aldrich); the cationic initiator: 2,2-azobis(N,N'-dimethylene isobutyramidine) dihydrochloride (ADIBA, Wako Chemical Gmbh); the cross-linking agent: poly(ethylene glycol) diacrylate (PEGDA 200, Polyscience) and the emulsifiers: dodecyltrimethylammonium bromide (DTAB), hexadecyltrimethylammonium bromide (HDTAB) and Tween 20, were commercially acquired.

The cationic emulsifier S7A13 (block copolymer with 7 units of styrene and 13 units of DMAEMA) was prepared according to the method shown in the following example.

### Example 1. S7A13 Synthesis: amphiphilic polystyrene (PSty) and 2-(dimethylamino)ethyl methacrylate (PDMAEMA) block copolymer.

### Materials

The following materials were used in this ARGET ATRP synthesis:
- Styrene (Aldrich) which was purified by means of distillation (5 mbar at 25°C);
- Dimethylethylamine methacrylate (Aldrich) which was also purified by means of distillation (2 mbar at 25°C);
- Copper(II) bromide (CuBr₂) (Aldrich) which was used as it was acquired (99-100% of purity).
- Ethyl-2-bromoisobutyrate (EBiB) (Aldrich) which was purified by means of distillation (3 mbar at 30°C) ; and
- tris[2-(dimethylamino)ethyl]amine (Me₆TREN) which was prepared as described in Macromolecules, 1998, 31, 5958 or in Inorg. Chem., 1966, 5, 41.

### Synthesis of the macroinitiator PSty

The macroinitiator PSty was prepared using ethyl 2-bromoisobutyrate (EBiB) as an initiator. The reaction was catalyzed by the CuBr₂/tris[2-(dimethylamino)ethyl]amine (Me₆TREN) complex under argon. Anisole was used as solvent and ascorbic acid was added in excess as reducing agent for reducing Cu(II) to Cu(I). The different components used in the synthesis of the macroinitiator PSty as well as the amounts of each of them are summarized in Table 1.

**Table 1. Components for the synthesis of the macroinitiator PSty-Br**

| | g | Equivalents | mol |
|---|---|---|---|
| Styrene | 59 | 9.57 | 0.57 |
| EBiB (98%) | 11 | 1 | 0.06 |
| CuBr₂ | 0.13 | 0.009 | 0.000567 |
| tris[2-(dimethylamino)ethyl]amine (Me₆TREN) | 1.3 | 0.076 | 0.004547 |
| Ascorbic acid | 1 | 10.2 | 0.005763 |
| Anisole | 43 | 35 | 0.40 |

The ligand (Me₆TREN) and CuBr₂ were mixed in a Schlenk tube with 5 grams of anisole to form the CuBr₂/tris[2-(dimethylamino)ethyl]amine complex. Argon was bubbled through the solution of the complex for 15 minutes while it was stirred. The ascorbic acid was dissolved in the remaining anisole and was added to a 250 mL three-neck flask. The styrene and the solution with the complex were transferred to the flask. 3 cooling cycles were performed on the reaction mixture and it was kept under argon atmosphere throughout the entire reaction. The temperature of the oil bath was set to 90°C and when the temperature of the reaction mixture was 90°C, the initiator EBiB was added for the purpose of starting polymerization. The reaction proceeded for 32 hours. 1 mL aliquots were taken during the reaction. The molecular weights were measured by means of size exclusion chromatography (SEC). The conversion was followed by means of gas chromatography using the reaction solvent as the internal standard. The final product was purified by means of precipitation in methanol. The polymer obtained was vacuum dried in an oven at 40°C for 24 hours. The product was characterized by means of SEC and MALDI-TOF and ¹H NMR.

The polymerization was suitably controlled, providing polymers with controlled molecular weights and low polydispersities. The final conversion was 80%. According to the SEC analysis, the average molecular weight in number (Mₙ) was 878 g/mol with a polydispersity of 1.2. MALDI-TOF analyses confirmed these results. The maximum theoretical content of copper in the polymer before purification was approximately 600 ppm. This content is very low if compared with the values for normal ATRP in which this content is around 40,000 ppm.

### Elongation of the chain of the macroinitiator PSty-Br with DMAEMA

The chain of the macroinitiator (PSty-Br) was elongated with DMAEMA for the purpose of providing a block copolymer. The macroinitiator (PSty-Br), DMAEMA and 95% by weight of anisole were added in a three-neck flask. 3 cooling cycles were applied to the reaction mixture and it was kept under argon atmosphere throughout the entire reaction. The reaction mixture was heated at 25°C. On the other hand, a mixture of the CuBr₂/tris[2-(dimethylamino)ethyl]amine (Me₆TREN) complex and 5% of anisole was degassed with argon for 15 minutes and was added to the previous reaction mixture. The components used for the chain elongation reaction of PSty-Br with DMAEMA, as well as their corresponding amounts, are shown in Table 2. The reaction proceeded for 24 hours. The final product was recrystallized in n-heptane to remove the PSty-Br which had not reacted. Even when the final product contained very small amounts of copper, the block copolymer was ultra-purified by means of dissolving 50 g of the polymer obtained in 1 L of water acidified with 10 mL of HCl_{aq} (33%). The solution was then neutralized by means of adding 20 g of a 25% NaOH solution in water. The precipitated block copolymer was recovered and then vacuum dried in an oven at 40°C for 24 hours. The composition of the block copolymer was determined by means of ¹H NMR.

**Table 2. Components for elongating the macroinitiator PSty-Br with DMAEMA**

| | g | equivalents | mol |
|---|---|---|---|
| DMAEMA | 76 | 8.54 | 0.43 |
| Psty-Br (Mₙ=878 g/Mol) | 43 | 1 | 0.050 |
| CuBr₂ | 0.11 | 0.010 | 0.0005 |
| tris[2-(dimethylamino)ethyl]amine (Me₆TREN) | 1.14 | 0.100 | 0.005 |
| Ascorbic acid | 0.87 | 9.9 | 0.005 |
| Anisole | 189 | 35 | 1.75 |

The chain elongation reaction showed a linear behavior when Mₙ was represented with respect to the conversion, which shows that the reaction proceeds under a controlled mechanism. The final conversion was 90% after 24 hours. Between 10 and 20% of the total amount of the macroinitiator PSty initially incorporated was recovered after the end of the reaction, showing that a significant fraction of the macroinitiator cannot be elongated. The final composition of the block copolymer according to the ¹H NMR spectrum comprised 13 units of DMAEMA and 7 units of styrene. After the final purification, it was confirmed by atomic spectroscopy that the total content of copper was virtually negligible.

### Quaternization of PSty₇-b-PDMAEMA₁₃

The block copolymer was dissolved by 4.5 times its weight in THF and CH₃I was added in two-fold molar excess. The reaction proceeded for 24 hours at room temperature. Polystyrene-b-poly(trimethyl ammonium ethyl methacrylate) (PSty-b-PTMAEMA) was recovered from the mixture after completing the reaction by means of removing the THF and subsequently washing the polymer with diethylether. The excess CH₃I used assured that the total amount of the DMAEMA groups was completely quaternized. ¹H NMR analyses were performed using D₂O as a solvent to show that all the amino groups were quaternized.

### Example 2: Synthesis of cationic nanogels stabilized with S7A13

These nanogels were synthesized in batches (discontinuously) in a reactor by means of emulsion copolymerization. VCL was used as the main monomer, PEGDA as the cross-linking agent, S7A13 as the emulsifier and ADIBA as the initiator.

The nanogel particles were prepared using different concentrations of emulsifier and initiator. Tables 3 and 4 show the recipes and reaction conditions used in the production of these nanogels varying the concentration of emulsifier and initiator, respectively.

**Table 3. Concentration variation of S7A13**

| Reagents | E2.5 | E5 | E10 | E15 | E20 |
|---|---|---|---|---|---|
| PEGDA (w/w% M) | 8 | 8 | 8 | 8 | 8 |
| S7A13 (w/w% M) | 2.5 | 5 | 10 | 15 | 20 |
| ADIBA (w/w% M) | 1.2 | 1.2 | 1.2 | 1.2 | 1.2 |
| * Reaction conditions: rpm= 300; | | | | | |
| reaction time= 5h; | | T= 70°C; | | * [VCL]=1 w/w % | |

**Table 4. Concentration variation of ADIBA**

| Reagents | E20I1.0 | | E20I0.5 | | E20I0.3 |
|---|---|---|---|---|---|
| PEGDA (w/w%) | 8 | | 8 | | 8 |
| S7A13 (w/w%) | 20 | | 20 | | 20 |
| ADIBA (w/w%) | 1.2 | | 0.6 | | 0.36 |
| * Reaction conditions: rpm= 300; | | | | | |
| reaction time=5h; | | T= 70°C; * | | [VCL] =1 w/w %. | |

### Example 3: Synthesis of cationic nanogels stabilized with a different type of emulsifier (S7A13, HDTAB, DTAB, or Tween 20)

These nanogels were synthesized in batches or discontinuously in a reactor by means of emulsion copolymerization. VCL was used as the main monomer, PEGDA as the cross-linking agent and ADIBA as the initiator. The nanogel particles were prepared using different emulsifiers: S7A13, HDTAB, DTAB, or Tween 20.

Table 5 shows the recipes and reaction conditions used in the production of these nanogels.

**Table 5**

| Reagents | E20 | H20 | D20 | NI20 |
|---|---|---|---|---|
| PEGDA (w/w% M) | 8 | 8 | 8 | 8 |
| S7A13 (mol% M) | 0.3 | - | - | - |
| HDTAB (mol% M) | - | 0.3 | - | - |
| DTAB (mol% M) | - | - | 0.3 | - |
| Tween 20 (mol% M) | - | - | - | 0.3 |
| ADIBA (w/w% M) | 1.2 | 1.2 | 1.2 | 1.2 |
| * Reaction conditions: rpm= 300; reaction time=5h; T= 70°C; * [VCL]=1 w/w % | | | | |

### Example 4. Effect of temperature of the medium on the average hydrodynamic diameter of the nanogel

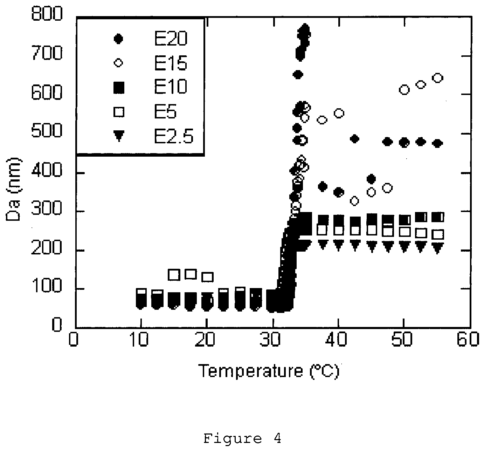

Figure 4 shows the response of the cationic nanogels, synthesized with different concentrations of S7A13, to the temperature changes of the dispersion medium. As can be seen, the nanogels are collapsed when the temperature of the medium is between 10-30°C. An instantaneous change or leap in size (average hydrodynamic diameter) then occurs which coincides with the phase transition temperature of the polyvinylcaprolactam (PVCL) homopolymer which would be obtained upon polymerizing the vinyl monomer (VCL) used in the synthesis as the main monomer alone. At low temperatures, sizes do not vary with the concentration of emulsifier. However, differences are observed when the temperature of the medium is above the transition temperature: the higher the concentration of emulsifier the larger the size of the nanogel.

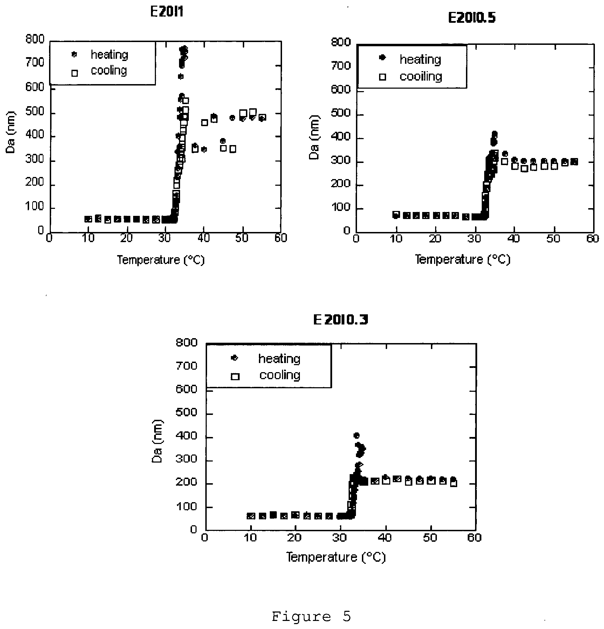

Next, Figure 5 shows the response of the cationic nanogels, synthesized by varying the concentration of initiator, to the temperature changes of the medium. It can be seen that the reduction of the concentration of initiator causes the reduction of the size of the nanogels at high temperatures. Furthermore, the behavior of the nanogel is reversible and the leap occurs both upon heating and upon cooling the medium in which the nanogel is dispersed.

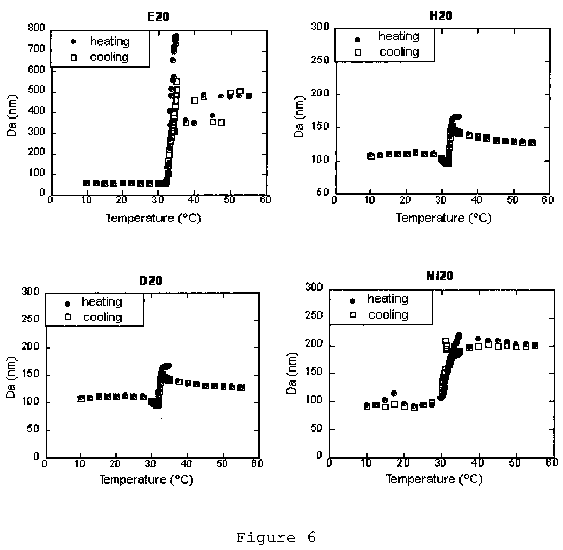

In terms of the effect of the type of emulsifier, Figure 6 show that the nanogels synthesized with both cationic emulsifier (HDTAB or DTAB) and non-ionic emulsifier (Tween 20) have the same behavior as nanogel E20 in which the amphiphilic block copolymer S7A13 has been used. It must be taken into account that even though a cationic emulsifier has not been used in the synthesis of nanogel NI20, it is also a cationic nanogel due to the positive charge from the initiator (ADIBA).

By comparing the diameters obtained with those of nanogel E20 used as a reference, it is observed that there are differences at both low and high temperatures. The nanogels synthesized using HDTAB or DTAB have the largest collapsed diameter (110 nm). The size of nanogel NI20 is 90 nm in the collapsed state, whereas the size of nanogel E20 is smaller, 50 nm.

In terms of the leap in the transition, it decreases when HDTAB or DTAB is used as emulsifier, swollen sizes less than those of nanogels E20 and NI20 being obtained. Therefore, the improvement provided by the use of the amphiphilic block copolymer S7A13 in the recipe with respect to the instantaneous leap from the collapsed state to the swollen state experienced by the nanogel particles as the temperature increases above the phase transition value of PVCL is evident.

It is still found that the behavior of the nanogel is reversible because the instantaneous leap from the collapsed state to the swollen state occurs both upon heating and upon cooling the medium in which the nanogel is dispersed.

### Example 5. Lyophilization-redispersion

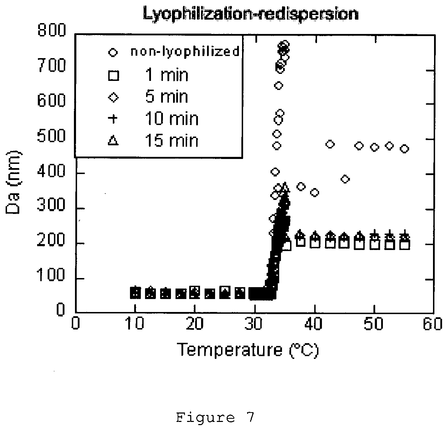

Figure 7 shows the behavior of the reference cationic nanogel (E20) in dispersion (non-lyophilized) and after its lyophilization and subsequent redispersion (resuspension time in minutes). The behavior of the nanogel lyophilized and then resuspended in water is the same as that of the non-lyophilized nanogel, i.e., collapsed at low temperatures and swollen at high temperatures. It is observed that the instantaneous leap occurs at the same temperature and that the collapsed sizes at low temperatures (10-30°C) remain identical, whereas the sizes corresponding to the swollen state at high temperatures are affected by lyophilization, being smaller than those the of the nanogel which has not been lyophilized.

## Claims

1. A biocompatible cationic nanogel comprising a polymer network, said polymer network consisting of polymer units interconnected with one another through a cross-linking agent, wherein said polymer network is obtainable by polymerizing N-vinylcaprolactam and a cross-linking agent in a dispersed medium, in the presence of a cationic initiator and a cationic or non-ionic emulsifier.

2. The nanogel according to claim 1, wherein said cross-linking agent is a difunctional monomer comprising at least two vinyl groups.

3. The nanogel according to claim 2, wherein the cross-linking agent is selected from ethylene glycol dimethacrylate (EGDMA), polydiethylene glycol dimethacrylate (PEGDMA), ethylene glycol diacrylate (EGDA), polydiethylene glycol diacrylate (PEGDA), ethylene glycol di(1-methacryloyloxy)ethyl ether, N,N'-methylenebisacrylamide (BA), a dextran with more than one vinyl group, and mixtures thereof.

4. The nanogel according to any of claims 1-3, wherein the cationic initiator is selected from the group consisting of 2,2-azobis(N,N'-dimethylene isobutyramidine) dihydrochloride (ADIBA), 2,2'-azobis[2-(2-imidazolin-2-yl)propane] disulfate dihydrate, 2,2'-azobisisobutyramidine dihydrochloride (AIBA), 2,2'-azobis(2-[1-(2-hydroxyethyl)-2-imidazolin-2-yl]propane} dihydrochloride and 2,2'-azobis(1-imino-1-pyrrolidino-2-ethylpropane) dihydrochloride.

5. The nanogel according to claim 4, wherein the cationic initiator is 2,2-azobis(N,N'-dimethylene isobutyramidine) dihydrochloride (ADIBA).

6. The nanogel according to any of claims 1-5, wherein the emulsifier is a cationic emulsifier.

7. The nanogel according to claim 6, wherein the cationic emulsifier is selected from hexadecyltrimethylammonium bromide, dodecyltrimethylammonium bromide and a quaternary ammonium block copolymer.

8. The nanogel according to claim 7, wherein the quaternary ammonium block copolymer is a styrene and 2-(dimethylamino) ethyl methacrylate block copolymer of formula: 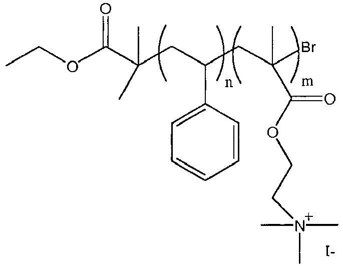 wherein n and m are integers comprised between 5 and 50.

9. The nanogel according to any of claims 1 to 8, further comprising a biologically active agent.

10. The nanogel according to any of claims 1 to 9 in the form of nanoparticles.

11. The nanogel according to any of claims 1 to 10 in a lyophilized form.

12. A pharmaceutical composition comprising a biocompatible cationic nanogel according to any of claims 1 to 11, and a pharmaceutically acceptable excipient, vehicle or adjuvant.

13. A method for obtaining a biocompatible cationic nanogel according to claim 1, which comprises polymerizing a composition consisting of N-vinylcaprolactam and a cross-linking agent in a dispersed medium, in the presence of a cationic initiator and a cationic or non-ionic emulsifier.

14. The method according to claim 13, wherein said polymerization is carried out in the presence of a biologically active agent.

15. The method according to claim 13, which further comprises contacting said biocompatible nanogel with a solution comprising a biologically active agent.

## Patentansprüche

1. Ein biokompatibles kationisches Nanogel, umfassend ein Polymernetzwerk, wobei das Polymernetzwerk aus Polymereinheiten besteht, die miteinander durch ein Vernetzungsmittel verbunden sind, wobei das Polymernetzwerk durch Polymerisieren *N*-Vinylcaprolactams und eines Vernetzungsmittels in einem dispergierten Medium, in Gegenwart eines kationischen Initiators und eines kationischen oder nichtionischen Emulgators, erhältlich ist.

2. Das Nanogel gemäß Anspruch 1, wobei das Vernetzungsmittel ein difunktionelles Monomer, welches mindestens zwei Vinylgruppen umfasst, ist.

3. Das Nanogel gemäß Anspruch 2, wobei das Vernetzungsmittel aus Ethylenglycoldimethacrylat (EGDMA), Polydiethylenglycoldimethacrylat (PEGDMA), Ethylenglycoldiacrylat (EGDA), Polydiethylenglycoldiacrylat (PEGDA), Ethylenglycol-di(1-methacryloyloxy)ethylether, N,N'-Methylenbisacrylamid (BA), einem Dextran mit mehr als einer Vinylgruppe, und Gemischen davon ausgewählt ist.

4. Das Nanogel gemäß einem der Ansprüche 1 bis 3, wobei der kationische Initiator aus der Gruppe bestehend aus 2,2-Azobis(N,N'-dimethylenisobutyramidin)-dihydrochlorid (ADIBA), 2,2'-Azobis[2-(2-imidazolin-2-yl)propan]-disulfatdihydrat, 2,2'-Azobisisobutyramidin-dihydrochlorid (AIBA), 2,2'-Azobis {2-[1-(2-hydroxyethyl)-2-imidazolin-2-yl]propan}-dihydrochlorid und 2,2'-Azobis(1-imino-1-pyrrolidino-2-ethylpropan)-dihydrochlorid ausgewählt ist.

5. Das Nanogel gemäß Anspruch 4, wobei der kationische Initiator 2,2-Azobis(N,N'-dimethylenisobutyramidin)-dihydrochlorid (ADIBA) ist.

6. Das Nanogel gemäß einem der Ansprüche 1 bis 5, wobei der Emulgator ein kationischer Emulgator ist.

7. Das Nanogel gemäß Anspruch 6, wobei der kationische Emulgator aus Hexadecyltrimethylammoniumbromid, Dodecyltrimethylammoniumbromid und einem quaternären Ammonium-Blockcopolymer ausgewählt ist.

8. Das Nanogel gemäß Anspruch 7, wobei das quaternäre Ammonium-Blockcopolymer ein Styrol- und 2-(Dimethylamino)ethylmethacrylat-Blockcopolymer der Formel: 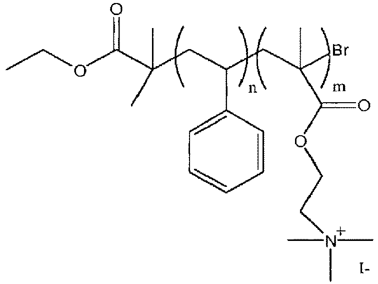 ist, wobei n und m zwischen 5 und 50 umgefasste ganze Zahlen sind.

9. Das Nanogel gemäß einem der Ansprüche 1 bis 8, ferner umfassend ein biologisch aktives Agens.

10. Das Nanogel gemäß einem der Ansprüche 1 bis 9 in Form von Nanopartikeln.

11. Das Nanogel gemäß einem der Ansprüche 1 bis 10 in einer lyophilisierten Form.

12. Eine pharmazeutische Zusammensetzung, umfassend ein biokompatibles kationisches Nanogel gemäß einem der Ansprüche 1 bis 11 und einen pharmazeutisch verträglichen Exzipienten, ein pharmazeutisch verträgliches Vehikel oder einen pharmazeutisch verträglichen Hilfsstoff.

13. Ein Verfahren zum Erhalten eines biokompatiblen kationischen Nanogels gemäß Anspruch 1, welches Polymerisieren einer Zusammensetzung, die aus *N-*Vinylcaprolactam und einem Vernetzungsmittel in einem dispergierten Medium besteht, in Gegenwart eines kationischen Initiators und eines kationischen oder nichtionischen Emulgators umfasst.

14. Das Verfahren gemäß Anspruch 13, wobei das Polymerisieren in Gegenwart eines biologisch aktiven Agens durchgeführt wird.

15. Das Verfahren gemäß Anspruch 13, welches ferner das In-Kontakt-Bringen des biokompatiblen Nanogels mit einer Lösung, welche ein biologisch aktives Agens umfasst, umfasst.

## Revendications

1. Un nanogel cationique biocompatible comprenant un réseau polymère, ledit réseau polymère étant constitué d'unités polymères reliées entre elles par l'intermédiaire d'un agent de réticulation, où ledit réseau polymère peut être obtenu par polymérisation en milieu dispersé de polymérisant N-vinylcaprolactame et d'un agent de réticulation, en présence d'un initiateur cationique et d'un émulsifiant cationique ou non-ionique.

2. Le nanogel selon la revendication 1, où ledit agent de réticulation est un monomère bifonctionnel comprenant au moins deux groupes vinyle.

3. Le nanogel selon la revendication 2, où l'agent de réticulation est sélectionné parmi diméthacrylate d'éthylène glycol (EGDMA), diméthacrylate de polydiéthylène glycol(PEGDMA), diacrylate d'éthylène glycol (EGDA), diacrylate de polydiéthylène glycol (PEGDA), éthylène glycol di(1-méthacryloyloxy)éthyl éther, N,N'-méthylènebisacrylamide (BA), un dextran avec plus d'un groupe vinyle, et des mélanges de ceux-ci.

4. Le nanogel selon l'une quelconque des revendications 1 à 3, où l'initiateur cationique est sélectionné parmi le groupe constitué de dichlorhydrate de 2,2-azobis(N,N'-diméthylène isobutyramidine) (ADIBA), dihydrate disulfate de 2,2'-azobis[2-(2-imidazolin-2-yl)propane], dichlorydrate de 2,2'-azobisisobutyramidine (AIBA), dichlorydrate de 2,2'-azobis{2-[1-(2-hydroxyéthyl)-2-imidazolin-2-yl]propane} et dichlorydrate de 2,2'-azobis(1-imino-1-pyrrolidino-2-éthylpropane).

5. Le nanogel selon la revendication 4, où l'initiateur cationique est du dichlorydrate de 2,2-azobis(N,N'-diméthylène isobutyramidine)(ADIBA).

6. Le nanogel selon l'une quelconque des revendications 1 à 5, où l'émulsifiant est un émulsifiant cationique.

7. Le nanogel selon la revendication 6, où l'émulsifiant cationique est sélectionné parmi du bromure d'hexadécyltriméthylammonium, du bromure de dodécyltriméthylammonium et un copolymère séquencé d'ammonium quaternaire.

8. Le nanogel selon la revendication 7, où le copolymère séquencé d'ammonium quaternaire est un styrène et un copolymère séquencé de méthacrylate de 2-(diméthylamino)éthyle de formule: 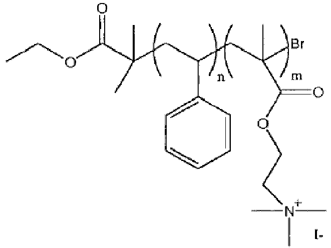 où n et m sont des entiers compris entre 5 et 50.

9. Le nanogel selon l'une quelconque des revendications 1 à 8, comprenant en outre un agent biologiquement actif.

10. Le nanogel selon l'une quelconque des revendications 1 à 9 sous forme de nanoparticules.

11. Le nanogel selon l'une quelconque des revendications 1 à 10 sous forme lyophilisée.

12. Une composition pharmaceutique comprenant un nanogel cationique biocompatible selon l'une quelconque des revendications 1 à 11, et un excipient, véhicule ou adjuvant pharmaceutiquement acceptable.

13. Un procédé d'obtention d'un nanogel cationique biocompatible selon la revendication 1, qui comprend la polymérisation en milieu dispersé d'une composition constituée de N-vinylcaprolactame et d'un agent de réticulation, en présence d'un initiateur cationique et d'un émulsifiant cationique ou non-ionique.

14. Le procédé selon la revendication 13, où ladite polymérisation est effectuée en présence d'un agent biologiquement actif.

15. Le procédé selon la revendication 13, qui comprend en outre la mise en contact dudit nanogel biocompatible avec une solution comprenant un agent biologiquement actif.
